# EUROPEAN PATENT APPLICATION

(11) **EP 3 015 848 A1**
(43) Date of publication of application: **04.05.2016**
(21) Application number: 14191071.1
(22) Date of filing: 30.10.2014
(51) Int. Cl.: G01N 21/47, A61B 5/024, A61B 5/1455, G01N 21/49, G01N 21/17

(54) **Apparatus and method for detecting light reflected from an object**

(71) Applicant: Nokia Technologies OY, 02610 Espoo (FI)
(72) Inventor: Lasarov, Harri, 02280 Espoo (FI)
(74) Representative: Nokia Corporation

(57) **Abstract**

Examples of the present disclosure relate to an apparatus and method for detecting light reflected from an object. The apparatus comprises: a light guiding element comprising a diffracting structure configured to direct incident light to an object; and an optical sensing element configured to detect light reflected from said object.

## Description

### TECHNOLOGICAL FIELD

Examples of the present disclosure relate to an apparatus and method for detecting light reflected from an object. Some examples, though without prejudice to the foregoing, relate to an apparatus and method for detecting light reflected from a user's body, e.g. skin, for determining biometric/physiological measurements, such as optically obtaining a volumetric measurement of an organ for a photoplethysmogram (PPG) to determine a user's heart rate.

### BACKGROUND

Physiological/biometric measurements such as a user's heart rate may be measured from tissue using an optical PPG sensor. A change in volume caused by a pressure pulse from each cardiac cycle of a user's heart may be detected by a pulse oximeter which detects changes in light absorption within the skin of a user. The skin is illuminated, for instance from a light emitting diode (LED), and an amount of light reflected is measured, for instance with a photodetector.

Conventional optical sensor systems are not always optimal. Light, for example from an LED, is typically emitted over a wide area and scattered all around which can reduce the amount of light that is reflected from an object and able to be detected by a photodetector. To compensate for such issues, brighter light sources and/or more power may be required in order to detect sufficient amount of reflective light to enable the determination of physiological/biometric measurements therefrom, such as heart rate measurements.

The listing or discussion of any prior-published document or any background in this specification should not necessarily be taken as an acknowledgement that the document or background is part of the state of the art or is common general knowledge. One or more aspects/examples of the present disclosure may or may not address one or more of the background issues.

### BRIEF SUMMARY

According to at least some but not necessarily all examples of the disclosure there is provided an apparatus comprising: a light guiding element comprising a diffracting structure configured to direct incident light to an object; and an optical sensing element configured to detect light reflected from said object.

The apparatus may further comprise a source of illumination. The light guiding element may be configured to provide a protective window/cover for the source of illumination. The light guiding element may further be configured to provide a protective window/cover for the optical sensing element.

Alternatively, a source of illumination may be provided as a first module and the optical sensing element may be provided as a second separate module. The light guiding element may be configured to provide a protective window/cover for the first module and a further light guiding element may be provided that is configured to provide a protective window/cover for the second module.

The apparatus may be provided as part of a device, such as a wearable device configured to at least partially enclose an appendage of a user, such as a user's wrist. The device may further configured for at least one of: wireless communication, mobile telephony, receiving user input, providing user output, portable use and wearable use.

According to at least some but not necessarily all examples of the disclosure there is provided a method comprising: using a light guiding element comprising a diffracting structure to direct incident light to an object; and detecting light reflected from said object.

According to at least some but not necessarily all examples of the disclosure there is provided an apparatus comprising means configured to perform the above method.

According to at least some but not necessarily all examples of the disclosure the apparatus may be configured for use as a sensor for determining one or more of: a biometric measurement, a physiological measurement, a photoplethysmogram measurement, a volumetric measurement, a blood oxygen saturation measurement, a cardiovascular measurement or heart rate measurement.

The examples of the present disclosure and the accompanying claims may be suitably combined in any manner apparent to one of ordinary skill in the art.

### BRIEF DESCRIPTION OF THE DRAWINGS

For a better understanding of various examples of the present disclosure that are useful for understanding the detailed description and certain embodiments of the invention, reference will now be made by way of example only to the accompanying drawings in which:
Figure 1 schematically illustrates an example of an apparatus according to the present disclosure;
Figure 2 schematically illustrates a further example of an apparatus according to the present disclosure;
Figure 3 schematically illustrates a yet further example of an apparatus according to the present disclosure;
Figure 4 schematically illustrates a yet further example of an apparatus according to the present disclosure; and
Figure 5 schematically illustrates a flowchart of a method according to the present disclosure.

### DETAILED DESCRIPTION

The Figures schematically illustrate an apparatus 100 comprising:
a light guiding element 101 comprising a diffracting structure 302 configured to direct incident light 102 to an object 103; and
an optical sensing element 105 configured to detect light reflected from said object 103.

The diffracting structure may, for example, be a diffractive optical element, a diffraction grating, a periodic structure/pattern or a series of diffraction lines/slits/grooves.

Certain examples of the present disclosure seek to provide an apparatus for re-orientating/focusing light to an object, such as a region of a user's body, so as to increase an amount of light that might be reflected from the object and detected by the optical sensing element. Increasing the amount of detected reflected light may improve the signal to noise ratio of the measurement of the reflected light. This may provide an advantage in that a lower brightness/luminous flux source of light might be employed for the incident light, e.g. one might be able to make use of an illumination source of a lower brightness/power than might otherwise have been required. This in turn may enable a reduction in power consumption of the apparatus.

Moreover, the use of a light guiding element with a diffracting structure and at least partly using diffraction to redirect the light may enable one or more surfaces of the light guiding structure to be devoid of user noticeable protrusions or recessions, which may otherwise have been required, for example were there instead a convex/concave lens used for focusing the light. Examples of the present disclosure may provide a light guiding element having a diffraction structure integrated into its surface wherein the diffractive structure has protrusions of the order of microns or nanometres. This is smaller than instances where a lens is used which might have a concave protrusion of the order of millimetres. Advantageously, this may reduce the size and thickness of the light guiding structure. Also, this may reduce restrictions in the macro/large-scale shape of the light guiding structure (such as reducing or avoiding the need for user noticeable/feelable protrusions/recessions in the light guiding element which might cause user discomfort particularly where the light guiding element is pressed against a part of the user's body for long periods of time). Also the light guiding structure may be used to form a protective cover/layer/window over the optical sensing element and/or form an external part of a housing for the apparatus, such as a window in the housing through which incident light and reflected light can pass.

Certain particular non-limiting examples of the present disclosure may be used for determining one or more of: a biometric measurement, a physiological measurement, a photoplethysmogram measurement, a volumetric measurement, a blood oxygen saturation measurement, a cardiovascular measurement or heart rate measurement. Advantageously, such examples may enable an improved measurement of, for instance heart rate signals by increasing the detection efficiency of light reflected from user tissue. Improved detection/measuring efficiency may mean that a more accurate reading might be obtained and/or less power might be consumed. Where the apparatus is provided as a portable/wearable device, this may lead to longer periods of use and longer battery life. Also, improved user comfort levels when wearing the device may mean that a user might wear the device for longer periods of time thereby enabling measurements over longer periods of time.

Examples of apparatuses according to the present disclosure will now be described with reference to the Figures. Similar reference numerals are used in the Figures to designate similar features. For clarity, all reference numerals are not necessarily displayed in all figures.

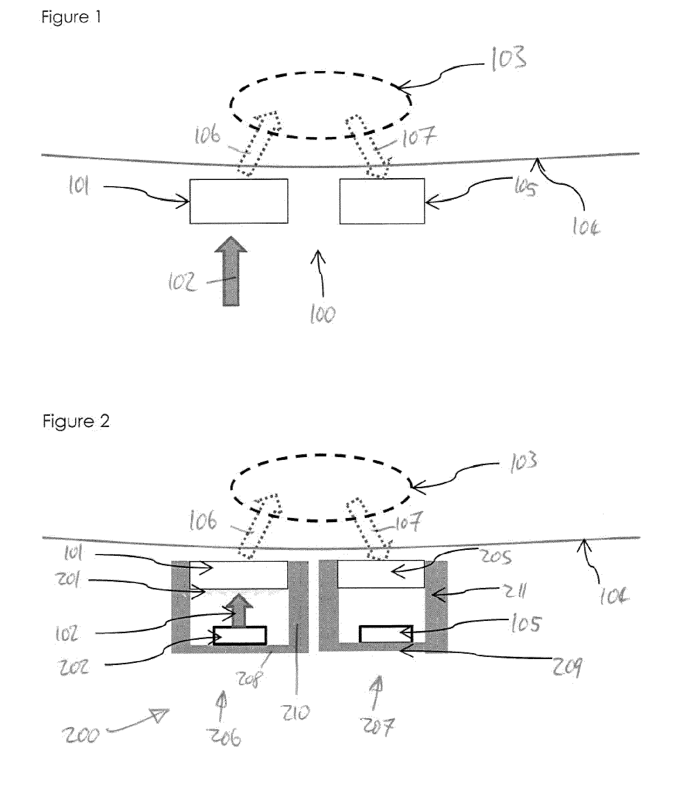

Figure 1 schematically illustrates an apparatus 100 according to the present disclosure. The apparatus 100 comprises a light guiding element 101, e.g. a light guide, and an optical sensing element 105, e.g. a light sensor, photo-detector or photodiode.

The light guiding element comprises a diffracting structure (not shown in Figure 1) and is configured so as to direct an incident light beam 102 to an object 103, such as a region 103 of a user's body 104. The incident light 102 is diffracted and reorientated/focused by the light guiding element towards the object/region 103 as indicated with redirected diffracted light 106. The redirected diffracted light 106 is reflected within the region 103 of the user's body, for example by tissue such as blood vessels and capillaries within the user's skin, and the reflected light 107 is detected by the optical sensing element 105. The optical sensing element, illumination source and light guiding element may be provided on a substrate, not least for example a printed wired board or flexible printed circuit board (not shown).

The apparatus 100 may be provided in a module. As used here `module' refers to a unit or apparatus that excludes certain parts/components that would be added by an end manufacturer or a user.

Certain particular non-limiting examples of the present disclosure may be used so as to provide photoplethysmogram (PPG) sensors for obtaining volumetric measurements of the user's tissue/blood vessels/capillaries within the region, particularly within the region of a user's skin.

Figure 2 shows a further apparatus 200 in which a source of illumination 202, such as a light emitting diode (LED), is used to generate the incident light 102 which is directed via a light guiding element 101 and its diffracting structure 201 so as to change its path to be re-orientated/redirected towards a particular region. Such directed light, as indicated by arrow 106, might then be reflected from the region, as indicated by arrow 107, and detected by the optical sensing element 105. The optical sensing element may be enclosed within a protective layer 205 which may, itself, comprise its own light guiding element (again which can comprise a diffractive structure - not shown) to re-direct/focus reflected light towards the optical sensing element 105. The light guiding element 101 may itself form a protective layer/window for the source of illumination 202.

Each of the light guiding elements 101 and 205 may direct light primarily via diffraction or may also be configured to guide light via refraction.

The apparatus may be provided as separate first and second modules, wherein the light guiding element 101 and source of illumination 202 are be provided as a first module 206, and the optical sensing element 105 and its protective layer/light guiding 205 are provided as a second module 207. For example, the light guiding element 101 and source of illumination 202 may be provided on a substrate 208 separate from a substrate 209 on which the optical sensing element 105 and its protective layer/light guiding 205 are provided. The light guiding element 101 and source of illumination 202 may be housed in a housing portion 210, having side walls and a lower/substrate section, that is separate from a housing portion 211 housing the optical sensing element 105 and its protective layer/light guiding 205.

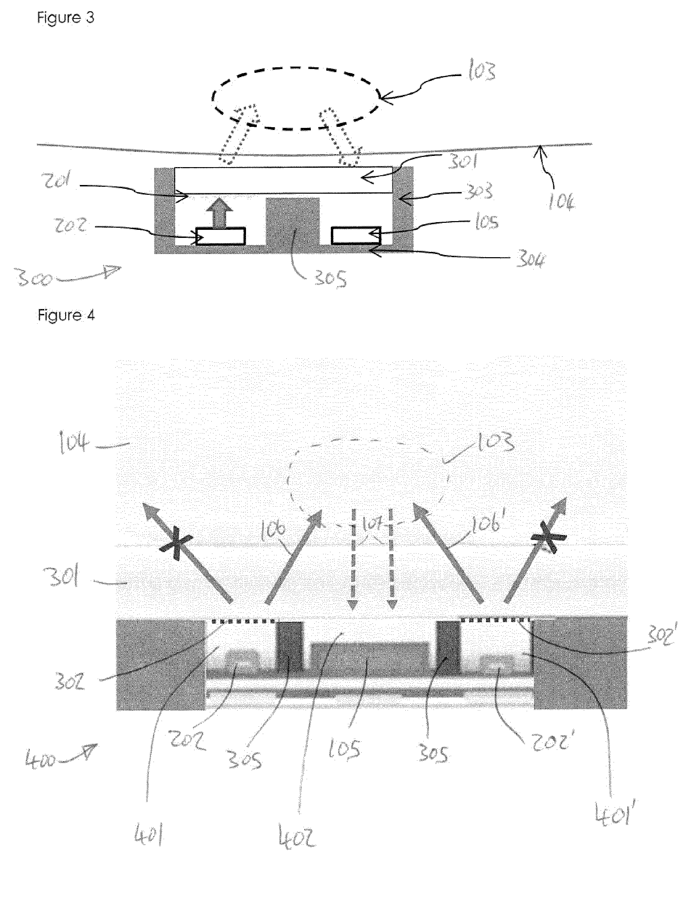

In some examples the light guiding element/protective layer 101 may be formed directly on the source of illumination 202. In some examples the light guiding element/protective layer 101 may be provided adjacent to the source of illumination 202. In some examples the light guiding element/protective layer 101 may be adjacent the source of illumination 202 such that the light guiding element/protective layer 101 is in contact with the source of illumination 202. In some examples there may be no gap or no air gap between the light guiding element/protective layer 101 and the source of illumination 202 (not shown in Figure 2 but shown in Figure 4). Likewise, light guiding element/protective layer 205 may be directly formed on the optical sensing element 105. In some examples the light guiding element/protective layer 205 may be provided adjacent to the optical sensing element 105. In some examples the light guiding element/protective layer 205 may be adjacent the optical sensing element 105 such that the light guiding element/protective layer 205 is in contact with the optical sensing element 105. In some examples there may be no gap or no air gap between the light guiding element/protective layer 205 and the optical sensing element 105 (not shown in Figure 2 but shown in Figure 4). In some examples, one or more optically transmissive materials may be interposed between the light guiding element and source of illumination/optical sensing element so as to fill in any air gaps. In other examples there may be air gaps provided between respective components.

Figure 3 shows a further example of an apparatus 300 according to the present disclosure in which a light guiding element 301 for a source of illumination 202 is shared with the optical sensing element 105, such that the light guiding element forms a protective window/cover for each of the source of illumination 202 and the optical sensing element 105.

The apparatus 300 comprises a housing structure 303 having side walls and a lower base/substrate section onto which the light source 202 and an optical sensing element 105 are provided. A dividing wall 305 is provided between the light source 202 and the optical sensing element 105 which serves to block the paths of any light coming directly from the light source 202 to the detector 105 without having passed through the light guiding element 301, for example by reflecting off an inner surface of the light guiding element 301. Alternatively, or in addition to providing such an optical barrier dividing wall, an anti-reflective coating may be provided to an underside/inner surface of the light guiding element so as to prevent reflection of light from the inner surface.

The source of illumination 202 emits light that is incident to the light guiding element 301 so as to be directed/reorientated to region 103. The light guiding element 301 comprises a diffractive structure 302. The diffractive structure may be provided on a surface of the light guiding element (e.g. a lower internal surface as shown, or an upper/external surface) and may be integrated therewith. An anti-reflective coating may be provided to an upper side/outer surface of the light guiding element.

The light guiding element may be formed from transparent materials such as not least for example optical grade plastic, glass or sapphire glass with high transparency. The light guiding element may form a protective window for the light source and/or sensor. The diffractive structure 302 may be integrated onto the protective window. The diffractive structure may be provided onto a surface via any suitable means, for example where the surface is plastic, the desired pattern may be replicated in moulding, casting, embossing, lasering, etching etc. Where the surface comprises other materials, such as glass or sapphire, etching, lasering or casting technologies may be used for example.

The diffractive structure is configured so as to assist in the redirection/focusing of a light beam 102 in a particular direction 106 such that a majority of the light is directed towards a particular region 103 or a particular focal point within the user's body. The diffracting structure may comprise one or more of a diffractive optical element, a periodic diffracting structure and/or diffracting line, slits or grooves. The period and/or shape and dimensions of a diffracting structure may be used to assist directing/focusing light to a particular region so as to concentrate the amount of light 106 able to be received at the region and thereby maximising the amount of light reflected 107 by the region and detected by the photodetector 105.

The diffracting structure 302 may be provided on at least a part of one or more of the major surfaces of the light guiding element, for example on at least a part of the inner/internal surface or the outer/external surface of the light guiding element. The diffractive structure may assist in reducing unwanted surface reflections and back scatterings.

The light guiding element 301 may be configured so as to extend over the optical sensing element thereby providing a protective layer/window over the optical sensing element. Such a protective layer/window comprising the diffractive structure may be curved or bendable, for example so as to conform to a shape of a user's body part to which the apparatus may be attached, e.g. wrist. The light guiding element 301 may be configured so as to direct/focus reflected light 107 onto the optical sensing element 105. An inner or outer surface of the light guiding element overlaying the optical sensing element may also be provided with a diffractive structure (not shown) so as to focus reflected light 107 onto the optical sensing element 105.

The incident light from a light source, such as an LED, may be visible light, such as green, blue, red light, or may comprise light from other non-visible parts of the electromagnetic spectrum, e.g. infrared light.

Figure 4 shows a further apparatus 400 comprising a plurality of light sources 202, 202' and an optical sensor 105. The apparatus also comprises a plurality of diffractive structures 302 and 302' which are appropriately arranged and configured so as to seek to maximise the amount of light that is directed towards a particular region 103 of a user's body 104 from their respective light source such that it might be reflected therefrom and detected by optical sensor 105. For example, the diffractive element 302 receives incident light from a light source 202 on a left hand side of the optical sensing element 105. The diffractive element 302 is configured so as to redirect/increase the amount of light heading towards a right hand side (as indicated with the rightward direction arrow) for refection onto the optical sensor 105 / photodiode area, and reduce the amount of light heading in a left hand side direction (as indicated with the leftward direction arrow with a cross therethrough). A reversed configuration is likewise provided for the other diffractive element 302'.

The apparatus 400 of Figure 4 is configured such that the light guide 301 and the light source 202, 202' are provided adjacent to each other. In some examples there is no air gap between the light guide 301 and the light sources 202, 202'. In other examples an air gap may be provided. For example, the light guide may surround the light source and be applied/formed directly thereon, e.g. such that the regions 401 and 401' form an integral part of the light guide. Alternatively, the regions 401 and 401' may comprise a material which is optically transmissive and optimally couples light from the light source to the light guide 301. Likewise, the apparatus may be configured such that there is no air gap between the light guide 301 and optical sensor 105, e.g. the region 402 may be an integral part of the light guide or an optical coupling medium/material.

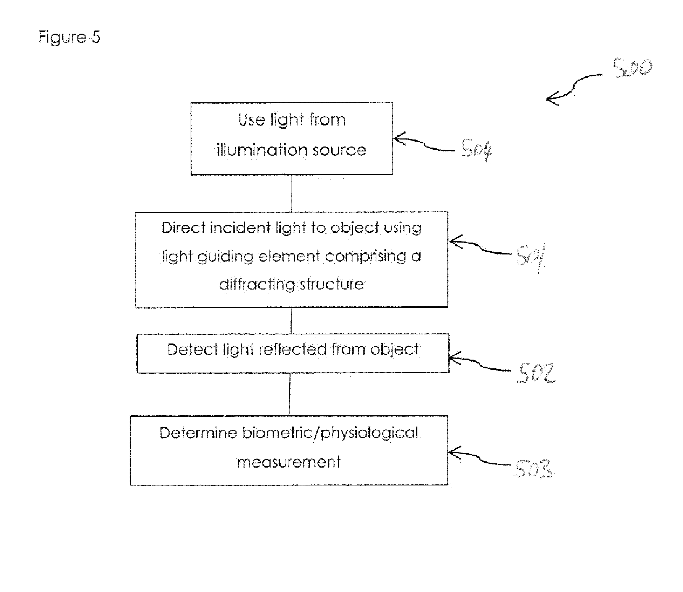

Figure 5 schematically illustrates a flowchart of a method 500 according to an example of the present disclosure. In block 501, incident light is directed to an object, e.g. a region of a user's body, using a light guiding element comprising a diffractive structure. The incident light may be at least in part from an illumination source as indicated in blocks 504.

In block 502 light reflected from the object is detected. In block 503 a biometric/physiological measurement is determined based on detected light. The physiological measurement may relate not least for example a photoplethysmogram measurement, a volumetric measurement, a blood oxygen saturation measurement, a cardiovascular measurement or a heart rate measurement.

It is to be appreciated that the blocks of the flowchart support both performing the specified functions as well as the provision of means configured to perform the specified function. Examples of the present disclosure relate to both a method and corresponding apparatus consisting of various modules or means that provide the functionality for performing the actions of the method. Thus, according to the present disclosure there may be provided an apparatus comprising means configured to cause the performance of the above described method.

The flowchart of figure 5 represents one possible scenario among others. The order of the blocks shown is not absolutely required, so in principle, the various blocks can be performed out of order. Not all of the blocks are essential. In certain examples, one or more of the blocks may be performed in a different order or overlapping in time, in series or in parallel. One or more of the blocks may be omitted or added or changed in some combination of ways. For example, block 504 may optionally be used, and block 503 may be omitted (as such functionality may be provided in a separate device, for example wherein a signal related to the amount of diffracted light reflected from the region that is detected is conveyed to a controller/processor remote of the apparatus for determining the physiological measurement). Whilst the redirecting/focusing of incident light by the light guiding element may be performed via diffraction within the diffracting structure of the light guiding element, the redirecting of the light may not solely be due exclusively to diffraction but could also be due to refraction occurring in the light guiding element, for example via a suitably configured light guiding element that may additionally provide refractive properties to direct/focus light to the region in addition to the diffraction occurring in the light guiding element. In this regard the light guiding structure may comprise one or more refractive optical microstructures in addition to one or more diffractive structures.

Furthermore, whilst a light guiding element comprising a diffracting structure is described, in certain examples, the light guiding element itself actually be, i.e. consist of, the diffracting structure. Such a light guiding element/diffracting structure may be provided on a surface of an optically transmissive medium such as glass or plastic optically transparent medium. Also, additional layers may be provided to the apparatus, such as anti-reflective coating layers to the light guiding element.

Certain examples of the present apparatus seek to provide beam shaping of incident light so as to increase luminous efficiency and enhance the efficiency of provision of light to a particular object/region. Such concentration of light provided to the object/region increases the amount of light that might be reflected from the object/region and able to be detected by the optical sensing element. Certain examples may also seek to provide beam shaping of reflected light so as to optimize collection of reflected light, i.e. enhance the efficiency of provision of reflected light to the optical sensing element. Such concentration of reflected light provided to the optical sensing element increases the amount of reflected light that might be detected.

Features described in the preceding description may be used in combinations other than the combinations explicitly described.

Although functions have been described with reference to certain features, those functions may be performable by other features whether described or not. Although features have been described with reference to certain examples, those features may also be present in other examples whether described or not.

It should be appreciated that modifications to the examples given can be made without departing from the scope of the invention as set out in the claims.

The term "comprise" is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one ..." or by using "consisting".

In this description, the wording "connect", "couple" and "communication" and their derivatives where used mean operationally connected/coupled/in communication. It should be appreciated that any number or combination of intervening components can exist (including no intervening components).

In this description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some or all other examples. Thus 'example', 'for example' or 'may' refers to a particular instance in a class of examples. A property of the instance can be a property of only that instance or a property of the class or a property of a sub-class of the class that includes some but not all of the instances in the class.

In this description, references to "a/an/the" [feature, element, component, means...] are to be interpreted as "at least one" [feature, element, component, means...] unless explicitly stated otherwise.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavouring in the foregoing specification to draw attention to those features of examples of the present disclosure believed to be of particular importance it should be understood that the applicant claims protection in respect of any patentable feature or combination of features hereinbefore referred to and/or shown in the drawings whether or not particular emphasis has been placed thereon.

## Claims

1. An apparatus comprising:
a light guiding element comprising a diffracting structure configured to direct incident light to an object; and
an optical sensing element configured to detect light reflected from said object.

2. The apparatus of claim 1, wherein the diffracting structure is integrated into a surface of the light guiding element.

3. The apparatus of any one or more of the previous claims, further comprising a source of illumination for generating at least a part of the incident light.

4. The apparatus of claim 3, wherein the light guiding element is configured to at least one or more of:
cover the source of illumination;
form a protective layer for the source of illumination; and
form an external part of a housing for the apparatus.

5. The apparatus of claims 3 or 4, wherein the apparatus is configured such that there is no air gap between the light guiding element and the source of illumination.

6. The apparatus of any one or more of the previous claims, wherein the light guiding element is further configured to at least one or more of:
cover the optical sensing element;
direct light reflected from the object to the optical sensing element;
form a protective layer for the optical sensing element; and
form an external part of a housing for the apparatus.

7. The apparatus of any one or more of previous claims 1 to 5, further comprising a second light guiding element, wherein the second light guiding element is configured to at least one or more of:
cover the optical sensing element;
direct light reflected from the object to the optical sensing element;
form a protective layer for the optical sensing element; and
form an external part of a housing for the apparatus.

8. The apparatus of claims 6 or 7, wherein the apparatus is configured such that there is no air gap between the light guiding element and the optical sensing element.

9. The apparatus of any one or more of the previous claims, wherein the object comprises a region of a user's body.

10. The apparatus of any one or more of the previous claims, wherein the apparatus comprises one or more of:
a support structure configured to at least partially enclose an appendage of a user; and
an attachment mechanism configured to attach the apparatus to a user's body.

11. The apparatus of any one or more of the previous claims, wherein the apparatus is configured for use in determining one or more of:
a biometric measurement, a physiological measurement,
a photoplethysmogram measurement, a volumetric measurement, a blood oxygen saturation measurement, a cardiovascular measurement or heart rate measurement.

12. A module or device comprising the apparatus of any one or more of previous claims 1 to 11.

13. A method comprising causing, at least in part, actions that result in:
using a light guiding element comprising a diffracting structure to direct incident light to an object; and
detecting light reflected from said object.

14. The method of claim 13, further comprising using, at least partly, an illumination source for the incident light.

15. The method of claim 14, further comprising determining one or more of:
a biometric measurement, a physiological measurement,
a photoplethysmogram measurement, a volumetric measurement, a blood oxygen saturation measurement, a cardiovascular measurement or heart rate measurement.
